(19) Europäisches Patentamt
European Patent Office
Office européen des brevets

(11) Veröffentlichungsnummer : **0 084 758**
**B1**

(12) # EUROPÄISCHE PATENTSCHRIFT

(45) Veröffentlichungstag der Patentschrift : **12.02.86**

(51) Int. Cl.⁴ : **C 07 D239/50**, A 01 N 43/54

(21) Anmeldenummer : **82810517.1**

(22) Anmeldetag : **01.12.82**

(54) **Als Schädlingsbekämpfungsmittel wirksame Pyrimidin Derivate.**

(30) Priorität : **07.12.81 CH 7801/81**

(43) Veröffentlichungstag der Anmeldung : **03.08.83 Patentblatt 83/31**

(45) Bekanntmachung des Hinweises auf die Patenterteilung : **12.02.86 Patentblatt 86/07**

(84) Benannte Vertragsstaaten : **AT BE DE FR IT NL**

(56) Entgegenhaltungen : **DE-A- 2 356 644**

(73) Patentinhaber : **CIBA-GEIGY AG**
**Klybeckstrasse 141**
**CH-4002 Basel (CH)**

(72) Erfinder : **Thummel, Rudolph C.**
**Route d'Alle 424**
**CH-2892 Courgenay (CH)**
Erfinder : **Fischer, Hanspeter, Dr.**
**Burggartenstrasse 14**
**CH-4103 Bottmingen (CH)**
Erfinder : **Burckhardt, Urs, Dr.**
**Rittergasse 29**
**CH-4051 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue insektizide und ektoparasitizide Mittel, die als aktive Komponente Amino-5-nitropyrimidin-Derivate oder deren Salze enthalten, und ihre Verwendung zur Bekämpfung von Insekten, darunter vor allem ektoparasitäre Insekten.

Die Amino-5-nitropyrimidine entsprechen der allgemeinen Formel Ia

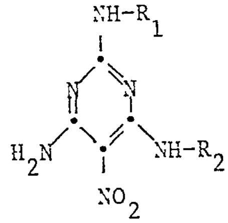

(Ia)

worin

$R_1$ die Isopropyl- oder die Cyclopropylgruppe und

$R_2$ Wasserstoff, die Isopropyl- oder die Cyclopropylgruppe bedeuten.

Unter Salzen der Amino-5-nitropyrimidin-Derivate der Formel Ia sind solche mit starken Mineralsäuren wie beispielsweise mit Halogenwasserstoffsäure oder Schwefelsäure zu verstehen.

Die Verbindungen der Formel Ia werden teilweise in allgemeiner Form von der DOS 2 356 644, die auf herbizide Wirkstoffe gerichtet ist, umfasst, jedoch ohne spezifische Nennung von Einzelverbindungen der Formel Ia.

Die Verbindungen der Formel Ia können nach an sich bekannter Methode (vgl. DE-OS 2 223 644 ; CH-PS 574 206 ; D.J. Brown, Heterocyclic Compounds, The Pyrimidines, Supplement I) hergestellt werden, indem man nucleofuge Gruppen in der 2-, 4-, und/oder 6-Stellung von 5-Nitropyrimidinen gegen gegebenenfalls substituierte Aminogruppen austauscht.

Als Ausgangsstoffe dienen 5-Nitropyrimidin-Derivate der Formeln IIa-IIe :

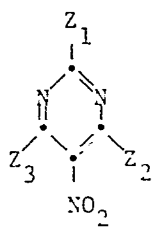 (IIa), 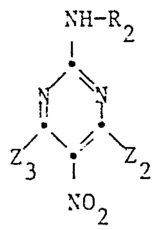 (IIb), 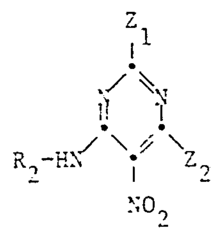 (IIc),

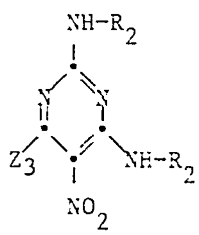 (IId), 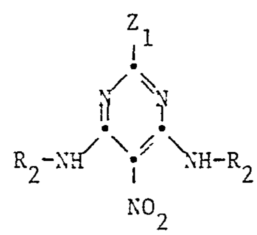 (IIe)

worin $R_1$ und $R_2$ die in der Formel Ia angegebenen Bedeutungen besitzen und die Reste $Z_1$, $Z_2$ und $Z_3$ nucleofuge Gruppen darstellen, vorzugsweise Halogenatome, insbesondere Chloratome, oder den Rest —X—$R_4$, in welchem X für Sauerstoff, Schwefel, die Sulfinyl- oder die Sulfonylgruppe und $R_4$ für Wasserstoff, Alkyl, Alkenyl, Alkinyl, substituiertes Alkyl, Cycloalkyl, Phenyl oder substituiertes Phenyl stehen. Die Gruppe —X—$R_4$ ist insbesondere $C_1$-$C_3$-Thioalkyl oder $C_1$-$C_3$-Alkoxy.

Beispiele solcher, grösstenteils aus der Literatur bekannter Ausgangsstoffe sind :

2,4,6-Trichlor-5-nitropyrimidin und 2-Methylthio-4,6-dichlor-5-nitropyrimidin.

Die Reaktion wird bei Temperaturen von − 60° bis + 160 °C in Gegenwart eines Lösungs- und/oder Verdünnungsmittels, gegebenenfalls in Gegenwart eines säurebindenden Mittels und bei Druckbedingungen von 0,1 MPa bis 10 MPa durchgeführt.

Als Lösungs- oder Verdünnungsmittel kommen für die Umsetzungen Wasser, Ketone wie Aceton oder Methyläthylketon, Aether und ätherartige Verbindungen wie Dioxan oder Tetrahydrofuran, aliphatische und aromatische Kohlenwasserstoffe und Halogenkohlenwasserstoffe, ferner Nitrile wie Acetonitril, N,N,dialkylierte Amide wie Dimethylformamid oder Sulfoxide wie Dimethylsulfoxid, sowie Gemische solcher Lösungsmittel untereinander in Betracht.

Als säurebindende Mittel sind für das Verfahren anorganische Basen wie Alkalimetall- und Erdalkalimetall-hydroxide, -hydrogencarbonate und -carbonate am besten geeignet. Es kommen aber

auch organische Basen, besonders tertiäre Amine wie Trisalkylamine, N,N-Dialkylaniline, Pyridin und Pyridinbasen in Frage. Ebenso kann die jeweilige Aminkomponente, im Ueberschuss eingesetzt, als säurebindendes Mittel dienen.

Als Ausgangsstoffe verwendete Nitropyrimidine können beispielsweise nach bekannten Verfahren (vgl. DE-OS 2 520 381 ; Houben-Weyl Bd. 10/1 S. 463-889) durch Nitrierung entsprechender Hydroxy-pyrimidine (6-Hydroxy-pyrimidine, 4,6-Dihydroxy-pyrimidine und 2,4,6-Trihydroxy-pyrimidin) mit Salpetersäure + Eisessig/Trifluoressigsäure, Nitriergemisch ($HNO_3$ + $H_2SO_4$) bei Temperaturen von − 50° bis 150 °C und anschliessender Einführung von Halogenatomen mit Hilfe von Phosphorhalogeniden wie $POCl_3$, $PCl_3$, $PBr_5$, $PCl_3$ oder mit Thionylchlorid bzw. Thionylbromid erhalten werden.

Ferner können die Verbindungen der Formel Ia analog zu bekannten Methoden (vgl. J. Org. Chem. *26* (1960) 455) durch Nitrierung der entsprechend substituierten 2,4,6-Triaminopyrimidine hergestellt werden.

Ueberraschenderweise wurde nun gefunden, dass die Verbindungen der Formel Ia eine ausgeprägte larvizide Wirkung auf Insektenlarven, vorzugsweise auf Dipterenlarven, besitzen. Sie können vor allem zur Bekämpfung von Hygieneschädlingen und von tierischen Ektoparasiten aus der Ordnung Diptera und den Familien Culcidae, Simuliidae, Tipulidae, Muscidae und Calliphoridae, sowie der Gattung Lucilia aus der Familie Calliphoridae eingesetzt werden.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen wie z. B. natürlichen oder regenerierten Stoffen, Lösungs- und/oder Dispergiermitteln.

Die Herstellung erfindungsgemässer Mittel erfolgt in an sich bekannter Weise durch inniges Vermischen und/oder Vermahlen von Wirkstoffen der Formel Ia mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgranulate, Imprägnierungsgranulate und Homogengranulate, Premix (Futterzusatz)

Flüssige Aufarbeitungsformen :

a) in Wasser dispergierbare Wirkstoffkonzentrate : Spritzpulver (wettable powders), Pasten, Emulsionen ;

b) Lösungen : Sprühmittel (Aerosole).

Zur Herstellung fester Aufarbeitungsformen (Stäubemittel, Streumittel) werden die Wirkstoffe mit festen Trägerstoffen vermischt. Als Trägerstoffe kommen zum Beispiel Kaolin, Talkum, Bolus, Löss, Kreide, Kalkstein, gemahlene Kunststoffe, gemahlene pflanzliche Produkte wie Getreidemehl, Aktivkohle etc. in Frage.

Granulate lassen sich sehr einfach herstellen, indem man einen Wirkstoff der Formel Ia in einem organischen Lösungsmittel löst und die so erhaltene Lösung auf ein granuliertes Mineral, z. B. Attapulgit, $SiO_2$, Granicalcium, Bentonit usw. aufbringt und dann das organische Lösungsmittel verdampft.

Granulate sind auch durch Kompaktieren des Trägermaterials mit den Wirk- und Zusatzstoffen und anschliessendes Zerkleinern erhältlich.

In Wasser dispergierbare Wirkstoffkonzentrate, d. h. Spritzpulver (wettable powders) Pasten und Emulsionskonzentrate stellen Mittel dar, die mit Wasser auf jede gewünschte Konzentration verdünnt werden können. Sie bestehen aus Wirkstoff, Trägerstoff, gegebenenfalls den Wirkstoff stabilisierenden Zusätzen, oberflächenaktiven Substanzen und Antischaumitteln und gegebenenfalls Lösungsmitteln. Die Spritzpulver (wettable powders) und Pasten werden erhalten, indem man die Wirkstoffe mit Dispergiermitteln und pulverförmigen Trägerstoffen in geeigneten Vorrichtungen bis zur Homogenität vermischt und vermahlt. Als Trägerstoffe kommen beispielsweise die vorstehend für die festen Aufarbeitungsformen erwähnten in Frage. In manchen Fällen ist es vorteilhaft, Mischungen verschiedener Trägerstoffe zu verwenden. Als Dispergatoren können beispielsweise verwendet werden : Kondensationsprodukte von sulfoniertem Naphthalin und sulfonierten Naphthalinderivaten mit Formaldehyd, Kondensationsprodukte des Naphthalins bzw. der Naphthalinsulfonsäuren mit Phenol und Formaldehyd sowie Alkali-, Ammonium- und Erdalkalisalze von Ligninsulfonsäure, ferner Alkylarylsulfonate, ditertiäre Aethylenglykole, fettsaure Alkali- und Erdalkalisalze und andere.

Die in der Formulierungstechnik gebräuchlichen Dispergatoren und Tenside sind u. a. in folgenden Publikationen beschrieben :

« Mc Cutcheon's Detergents and Emulsifiers Annual » MC
Publishing Corp., Ringwood New Jersey, 1980
Sisely and Wood, « Encyclopedia of Surface Active Agents »,
Chemical Publishing Co., Inc. New York, 1980.

Ferner können die erfindungsgemässen Mittel in Form von Lösungen angewendet werden. Hierzu wird der Wirkstoff bzw. werden mehrere Wirkstoffe der allgemeinen Formel Ia in geeigneten organischen Lösungsmitteln oder Lösungsmittelgemischen zugemischt. Als organische Lösungsmittel können aliphatische und aromatische Kohlenwasserstoffe, deren chlorierte Derivate, Alkylnaphthaline, Mineralöle allein oder als Mischung untereinander verwendet werden. Die Wirkstoffe der Formel I können beispielsweise wie folgt formuliert werden :

Stäubemittel :

Zur Herstellung eines a) 0,5 %igen und b) 2 %igen Stäubemittels werden die folgenden Stoffe verwendet :

a) 0,5 Teile Wirkstoff Nr. 1
99,5 Teile Talkum
b) 2 Teile Wirkstoff Nr. 4
1 Teil hochdisperse Kieselsäure
97 Teile Talkum.

Die Wirkstoffe werden mit den Trägerstoffen vermischt und vermahlen.

Granulat :

5 Teile Wirkstoff werden in einem Lösungsmittel wie Methylenchlorid gelöst und mit
2 Teilen Polyäthylenglykol (« Carbowachs ») gemischt.
Mit der Mischung werden
91,5 Teile Calciumcarbonat imprägniert und
1,5 Teile gefällte Kieselsäure zugemischt und anschliessend wird das Lösungsmittel verdampft.

Spritzpulver :

50 Teile Wirkstoff werden mit
5 Teilen eines Dispergators z. B. Ligninsulfonsäure-Natriumsalz,
5 Teilen eines Netzmittels z. B. Dibutylnaphthalinsulfonsäure,
10 Teilen Kieselsäure und
30 Teilen Kaolin gemischt und fein vermahlen.

Emulgierbares Konzentrat :

20 Teile Wirkstoff Nr. 1 werden mit
20 Teilen Emulgator z. B. Mischung von Alkylarylpolyglykoläther mit Alkylarylsulfonaten und
60 Teilen Lösungsmittel gemischt, bis die Lösung vollkommen homogen ist. Dieses Konzentrat gibt mit Wasser eine Emulsion jeder gewünschten verdünnten Konzentration.

Premix (Futterzusatz) :

0,25 Teile Wirkstoff Nr. 1 und
4,75 Teile sekundäres Calcium-Phosphat oder Kaolin, Aerosil oder kohlensaurer Kalk werden mit
95 Teilen eines Futtermittels wie z. B. Kaninchenfutter homogen vermischt.

Sprühmittel :

Zur Herstellung eines 3 %igen Sprühmittels werden die folgenden Bestandteile verwendet :
3 Teile Wirkstoff
97 Teile Kerosen.

Den beschriebenen Mitteln lassen sich andere biozide Wirkstoffe oder Mittel beimischen. So können die neuen Mittel ausser den genannten Verbindungen der allgemeinen Formel Ia zum Beispiel Insektizide zur Verbreiterung des Wirkungsspektrums enthalten.

Die erfindungsgemässen Mittel, bzw. die enthaltenen Wirkstoffe entfalten ihre entwicklungshemmende Wirkung in erster Linie gegen Larven oder Puppen von Insekten, vorzugsweise der Ordnung Diptera.

Zur Bekämpfung von auf oder in den Tieren parasitierenden Insekten und ihrer Entwicklungsstadien eignen sich solche Formulierungen der erfindungsgemässen Mittel, die eine verzögerte Freisetzung der Wirkstoffe der Formel Ia im Verdauungstrakt der Tiere bewirken. Darüber hinaus wird durch die orale Verabreichung von Verbindungen der Formel Ia an Tiere die Entwicklung von Insekten in den tierischen Ausscheidungen (Faces flora) verhindert und damit für die Freihaltung der Vieh-Stallungen von Insekten und Ihren Entwicklungsstadien wie beispielsweise Larven gesorgt.

Herstellung von Wirkstoffen

Beispiel 1

a) Herstellung eines Zwischenprodukts

Zu einer Lösung von 100 g 4,6-Dichlor-2-methylthio-5-nitropyrimidin in 500 ml Dioxan werden

während 6 Stunden $NH_3$-Gas im Ueberschuss eingeleitet. Nach dem Abkühlen verdünnt man mit 500 ml Wasser, filtriert und reinigt den Rückstand durch Aufkochen in Dioxan. Auf diese Weise erhält man das 4,6-Diamino-2-methylthio-5-nitropyrimidin mit einem Schmelzpunkt von 260 bis 262 °C in einer Ausbeute von 75 % der Theorie.

b) 20 g 4,6-Diamino-2-methylthio-5-nitropyrimidin werden im Druckrohr mit 20 g Cyclopropylamin und 40 ml Dioxan während 6 Stunden auf 140 °C erhitzt. Nach dem Erkalten wird das Reaktionsgemisch mit Wasser verdünnt und filtriert. Der Rückstand ergibt nach der Reinigung durch Kristallisation aus Aethanol das 4,6-Diamino-2-cyclopropylamino-5-nitropyrimidin mit einem Schmelzpunkt von 180 bis 183 °C in einer Ausbeute von 50 % der Theorie [Verb. Nr. 1].

Beispiel 2

a) Herstellung eines Zwischenprodukts

Zu einer Suspension von 10 g des Zwischenprodukts gemäss Beispiel 1a) in 50 ml Tetrahydrofuran (THF) wird bei Raumtemperatur eine Lösung von 10 g 3-Chlorperbenzoesäure in 40 ml THF getropft. Nach Zugabe weiterer 70 ml THF wird 3 Stunden gerührt. Die gebildeten Kristalle werden abgesaugt und bei 50 °C in 100 ml Dimethylformamid gelöst. Durch vorsichtige Zugabe von 200 ml Aethanol fällt reines 4,6-Diamino-2-methylsulfinyl-5-nitro-pyrimidin aus (68 % der Theorie), Schmelzpunkt 247 °C.

b) Zu einer Suspension von 2,2 g des gemäss 2a erhaltenen Zwischenprodukts in einem Gemisch aus 15 ml Wasser und 15 ml Dioxan werden 6 g Cyclopropylamin getropft. Nach einstündigem Erhitzen auf 100 °C und Abkühlen wird filtriert. Aus dem Filtrat erhält man durch Einengen 4,6-Diamino-2-cyclopropylamino-5-nitropyrimidin (90 % der Theorie), Schmelzpunkt 186 bis 187 °C [Verb. Nr. 1].

Auf analoge Art oder nach einer der weiter oben angegebenen Methoden lassen sich auch die folgenden Verbindungen Nr. 2 bis 5 herstellen.

| Nr. | $R_1$ | $R_2$ | Schmelzpunkt °C |
|---|---|---|---|
| 1 | 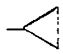 | H | 186 bis 187 |
| 2 | $-CH(CH_3)_2$ | H | 123 bis 127 |
| 3 | $-CH(CH_3)_2$ | $-CH(CH_3)_2$ | fest |
| 4 | 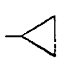 | $-CH(CH_3)_2$ | 178 - 181 |
| 5 | $-CH(CH_3)_2$ | 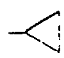 | fest |

Verbindungen der Formel Ia, in denen $R_1$ den Cyclopropylrest bedeutet, sind bisher nicht bekannt. Demzufolge sind die beiden Verbindungen 2-Cyclopropylamino-4,6-diamino-5-nitropyrimidin [Verb. Nr. 1] und 4-Amino-2-cyclopropylamino-6-isopropylamino-5-nitropyrimidin [Verb. Nr. 4] neu und stellen einen weiteren Gegenstand vorliegender Erfindung dar.

Als bevorzugtes Insektizid sind das 2-Cyclopropylamino-4,6-diamino-5-nitropyrimidin und seine Salze anzusehen.

Biologische Prüfung auf Schmeissfliegen

Beispiel 3

Die zu prüfenden Substanzen werden aus einem Spritzpulver oder einem Emulsionskonzentrat durch Verdünnen mit Wasser auf eine der gewünschten Wirkstoffkonzentrationen im Bereich von 100 ppm bis 1,5 ppm eingestellt (Verdünnungsfaktor 2).

a) Wirkung gegen Lucilia sericata (OP-sensibel)

Frisch abgelegte Eier werden je in Portionen von 30 bis 50 Stück in ein Reagenzglas gegeben, in dem sich ein Gemisch aus 4 ml Nährmedium und 1 ml Wirkstoff-Lösung befindet. Die Gläser werden mit einem Wattebausch verschlossen und 4 Tage bei 30 °C bebrütet. Nach diesem Zeitraum schlüpfen aus unbehandelten Eiern der Kontrollproben ca. 1 cm lange Larven (L-3). Die behandelten Proben werden auf tote oder moribunde Eier bzw. Larven ausgewertet. Als Grenzkonzentration dient die tiefste noch voll wirksame Wirkstoff-Konzentration.

b) Wirkung gegen Lucilia cuprina (OP-resistent)

Die Testmethodik gleicht der unter a) angegebenen.
In beiden Versuchen waren die Verbindungen Nr. 2 bis 4 in höheren Konzentrationen voll wirksam. Die Verbindung Nr. 1 zeigte bis zu 1,5 ppm volle Wirksamkeit (keine lebenden Larven).

**Patentansprüche**

1. Mittel zur Bekämpfung von Schädlingen, dadurch gekennzeichnet, dass es neben Träger- und/oder Zuschlagstoffen als aktive Komponente eine Verbindung der Formel Ia

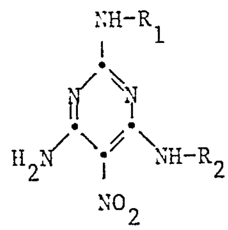

(Ia)

worin
$R_1$ die Isopropyl- oder die Cyclopropylgruppe und
$R_2$ Wasserstoff, die Isopropyl- oder die Cyclopropylgruppe bedeuten, oder ein Salz davon, enthält.
2. Mittel gemäss Anspruch 1 enthaltend als aktive Komponente 2-Cyclopropylamino-4,6-diamino-5-nitropyrimidin oder eines seiner Salze.
3. Eine Verbindung der Formel Ia'

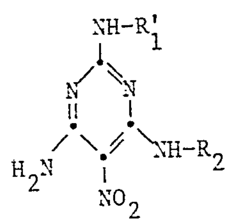

(Ia')

worin $R_1'$ die Cyclopropylgruppe und $R_2$ Wasserstoff, die Isopropyl- oder die Cyclopropylgruppe bedeuten.
4. Die Verbindungen 2-Cyclopropylamino-4,6-diamino-5-nitropyrimidin und 4-Amino-2-cyclopropylamino-6-isopropylamino-5-nitropyrimidin gemäss Anspruch 3.
5. Verfahren zur Herstellung der Verbindungen der Formel Ia gemäss Anspruch 1, dadurch gekennzeichnet, dass man Verbindungen der Formeln IIa bis IIe

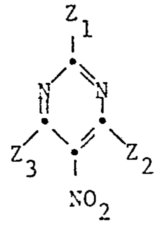 (IIa), 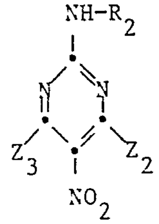 (IIb), 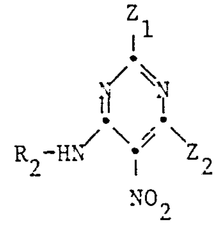 (IIc),

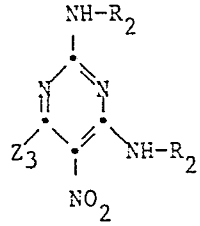 (IId), 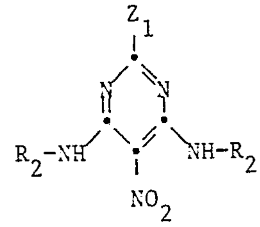 (IIe)

worin $R_1$ und $R_2$ die im Anspruch 1 angegebenen Bedeutungen besitzen und die Reste $Z_1$, $Z_2$ und $Z_3$ Halogenatome oder den Rest —X—$R_4$ darstellen, in welchem X Sauerstoff, Schwefel, die Sulfinyl- oder die Sulfonylgruppe und $R_4$ Wasserstoff, Alkyl, Alkenyl, Alkinyl, substituiertes Alkyl, Cycloalkyl, Phenyl oder substituiertes Phenyl sind, bei Temperaturen von − 60° bis + 160 °C in Gegenwart von Lösungs- und/oder Verdünnungsmitteln und gegebenenfalls in Gegenwart von säurebindenden Mitteln mit $NH_3$ oder mit $NH_2$—$R_1$ oder $NH_2$—$R_2$, in denen $R_1$ und $R_2$ die im Anspruch 1 angegebenen Bedeutungen besitzen, umsetzt.

6. Verwendung einer Verbindung der Formel Ia gemäss Anspruch 1 zur Bekämpfung von Schädlingen.

7. Verwendung gemäss Anspruch 6 zur Bekämpfung von Insekten der Ordnung Diptera.

8. Verwendung gemäss Anspruch 6 zur Bekämpfung von Ektoparasiten.

9. Verwendung gemäss Anspruch 6 zur Bekämpfung von Insekten der Gattung Lucilia.

10. Verwendung gemäss Anspruch 6, wobei als Wirkstoff 2-Cyclopropylamino-4,6-diamino-5-nitropyrimidin oder eines seiner Salze eingesetzt wird.

**Claims**

1. A composition for controlling pests, which composition contains as active ingredient a compound of the formula Ia

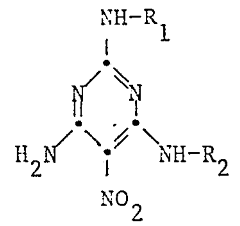

(Ia)

wherein

$R_1$ is the isopropyl or cyclopropyl group, and

$R_2$ is hydrogen, or the isopropyl or cyclopropyl group, or a salt thereof, together with carriers and/or other additives.

2. A composition according to claim 1, which contains as active ingredient 2-cyclopropylamino-4,6-diamino-5-nitropyrimidine, or one of the salts thereof.

3. A compound of the formula Ia'

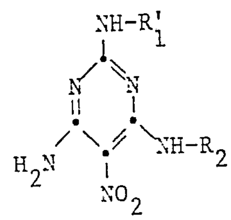

(Ia')

wherein $R_1'$ is the cyclopropyl group, and $R_2$ is hydrogen, or the isopropyl or cyclopropyl group.

4. The compounds 2-cyclopropylamino-4,6-diamino-5-nitropyrimidine and 4-amino-2-cyclopropylamino-6-isopropylamino-5-nitropyrimidine according to claim 3.

5. A process for the preparation of a compound of the formula Ia according to claim 1, which process comprises reacting a compound of any one of the formulate IIa to IIe

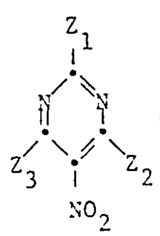

(IIa),

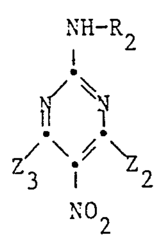

(IIb),

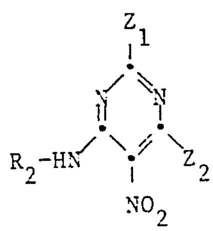

(IIc),

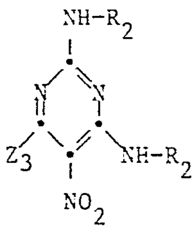 (IId), 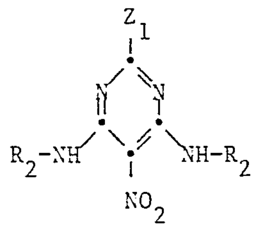 (IIe)

wherein $R_1$ and $R_2$ are as defined in claim 1, and $Z_1$, $Z_2$ and $Z_3$ are halogen atoms or the group —X—$R_4$ in which X is oxygen, sulfur, or the sulfinyl or sulfonyl group, and $R_4$ is hydrogen, alkyl, alkenyl, alkynyl, substituted alkyl, cycloalkyl, phenyl or substituted phenyl, at a temperature in the range from − 60° to + 160 °C, in the presence of a solvent and/or diluent and optionally in the presence of an acid acceptor, with $NH_3$ or with $NH_2$—$R_1$ or $NH_2$—$R_2$, in which $R_1$ and $R_2$ are as defined in claim 1.

6. A method of controlling pests, which method comprises applying thereto or to the locus thereof an effective amount of a compound of the formula Ia according to claim 1.

7. A method according to claim 6 for controlling insects of the order Diptera.

8. A method according to claim 6 for controlling ectoparasites.

9. A method according to claim 6 for controlling insects of the species Lucilia.

10. A method according to claim 6, which method comprises the use, as active ingredient, of 2-cyclopropylamino-4,6-diamino-5-nitropyrimidine or of one of the salts thereof.

## Revendications

1. Agent de lutte antiparasitaire, caractérisé en ce qu'il contient comme composant actif, outre des supports et/ou additifs, un composé de formule Ia

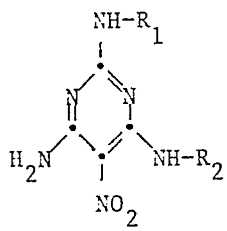 (Ia)

où

$R_1$ représente le groupe isopropyle ou cyclopropyle et

$R_2$ représente un hydrogène, le groupe isopropyle ou cyclopropyle ou un de ses sels.

2. Agent selon la revendication 1 contenant comme composant actif la 2-cyclopropylamino-4,6-diamino-5-nitropyrimidine ou un de ses sels.

3. Composé de formule Ia'

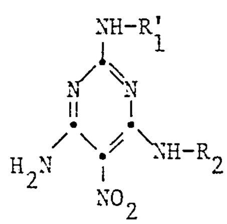 (Ia')

où $R_1'$ représente le groupe cyclopropyle et $R_2$ un hydrogène, le groupe isopropyle ou cyclopropyle.

4. Les composés 2-cyclopropylamino-4,6-diamino-5-nitropyrimidine et 4-amino-2-cyclopropylamino-6-isopropylamino-5-nitropyrimidine selon la revendication 3.

5. Procédé de préparation des composés de formule Ia selon la revendication 1, caractérisé en ce que l'on fait réagir des composés de formule IIa-IIe

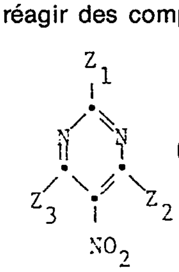 (IIa), 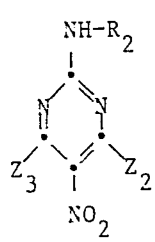 (IIb), 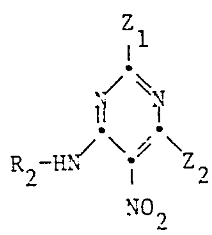 (IIc),

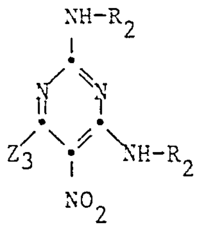 (IId), 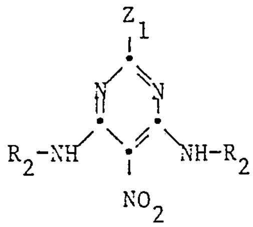 (IIe)

où $R_1$ et $R_2$ ont les significations données dans la revendication 1 et les radicaux $Z_1$, $Z_2$ et $Z_3$ représentent des atomes d'halogène ou le radical —X—$R_4$, où X est un oxygène, un soufre, le groupe sulfinyle ou le groupe sulfonyle et $R_4$ représente hydrogène, alcoyle, alcényle, alcynyle, alcoyle substitué, cycloalcoyle, phényle ou phényle substitué, à des températures allant de $-$ 60° à $+$ 160 °C en présence de solvants et/ou de diluants et éventuellement en présence d'agents liants d'acides avec $NH_3$ ou avec $NH_2$—$R_1$ ou $NH_2$—$R_2$ où $R_1$ et $R_2$ ont les significations données dans la revendication 1.

6. Application d'un composé de formule Ia selon la revendication 1 pour combattre les parasites.

7. Application selon la revendication 6 pour combattre les insectes de l'ordre des diptères.

8. Application selon la revendication 6 pour combattre les ectoparasites.

9. Application selon la revendication 6 pour combattre les insectes du genre Lucilia.

10. Application selon la revendication 6, où l'on utilise comme matière active la 2-cyclopropylamino-4,6-diamino-5-nitropyrimidine ou l'un de ses sels.